# EUROPEAN PATENT APPLICATION

(11) **EP 2 676 666 A1**
(43) Date of publication of application: **25.12.2013**
(21) Application number: 12172847.1
(22) Date of filing: 20.06.2012
(51) Int. Cl.: A61K 31/5025, A61K 51/04, A61P 9/14

(54) **Compounds for use in diagnosis or therapy of vulnerable atherosclerotic plaques**

(71) Applicant: Eidgenössische Technische Hochschule Zürich (ETH), 8092 Zürich (CH); Universität Zürich, 8006 Zürich (CH)
(72) Inventor: Müller, Adrienne, CH-8400 Winterthur (CH); Kraemer, Stefanie, CH-8049 Zürich (CH); Ametamey, Simon Mensah, CH-8045 Zürich (CH); Schibli, Roger, CH-5400 Baden (CH); Mu, Linjing, CH-5600 Lenzburg (CH)
(74) Representative: Schmauder & Partner AG Patent- & Markenanwälte VSP

(57) **Abstract**

A compound for use in diagnosis or therapy of vulnerable atherosclerotic plaques in a human patient comprises an active moiety capable of binding to CD80 and/or CD86 and further contains a radioisotope.

## Description

### Field of the Invention

The present invention generally relates to compounds for use in diagnosis or therapy of vulnerable atherosclerotic plaques.

### Back^{q}round of the Invention

Atherosclerosis is a pathological remodeling of the arteries which is a major cause of morbidity and mortality all over the world. This pathology is the basis of myocardial infarction, stroke and peripheral artery disease.

Atherosclerosis can be considered as an unusual form of chronic inflammation occurring within the arterial wall, which leads to the recruitment of immune cells, immune response, inflammation and apoptosis. Some atherosclerotic plaques are more likely to rupture than others. Those that are prone to rupture are designated "vulnerable" plaques. The rupture of a vulnerable plaque in a carotid or coronary artery often leads to stroke or fatal myocardial infarction without prior symptoms. Plaque rupture can be prevented by surgery (removal of the vulnerable plaque) or other therapeutic intervention. However, up to now there is no routine method to detect vulnerable plaques. Plaques that are less prone to rupture are known as "stable" plaques. These plaques are in most cases not immediately life-threatening and have a high prevalence in the elderly population. Vulnerability is associated with a thin fibrous cap (in contrast to a thicker cap in stable plaques), a large lipid core of the plaque with partial necrosis, a high density of plaque microvessels and a high infiltration with macrophages and T cells [1].

So far atherosclerotic plaques can be detected by a broad range of invasive and non-invasive imaging modalities.

¹⁸F-labeled fluorodeoxyglucose (FDG) is currently the most validated tracer for imaging of plaque inflammation [4]. However, high accumulation in myocardium precludes imaging of coronary vulnerable plaques.

Besides glucose metabolism, numerous other metabolic and signaling pathways associated with plaque vulnerability have been targeted with nuclear imaging modalities. These include the oxidation and accumulation of LDL with ¹²⁵iodine-labeled oxidation-specific antibodies [5], matrix metalloproteinase activity with ^{99m}technetium-labeled matrix metalloprotease inhibitor [6], macrophage apoptosis with ^{99m}technetium-labeled annexin-V [7-9], and finally monocyte recruitment with either ^{99m}technetium-labeled monocyte chemotactic protein-1 [10], an ¹⁸F-labeled peptide, which can be internalized by endothelial cells through VCAM-1-mediated binding [11], or the alpha(v)beta(3)-integrin-targeted PET tracer, ¹⁸F-galacto-RGD [12]. However, none of these methods was shown to unambiguously identify vulnerable plaques in human coronaries and carotids.

Atherosclerotic plaques, containing inflammatory infiltrates, predominantly consist of monocytes/macrophages, activated T cells, B cells and dendritic cells (DCs). The expression of co-stimulatory molecules that provide accessory signals during antigen-specific activation is a prerequisite for disease development. T-lymphocyte activation antigens CD80 (activation B7-1 antigen) and CD86 (activation B7-2 antigen) are closely related molecules expressed on antigen-presenting cells (B cells, dendritic cells) that provide co-stimulatory signals for Tcell activation [13]. T cell-specific surface glycoprotein CD28 and cytotoxic T-lymphocyte protein 4 (CTLA-4) can be detected on a small amount of T cells [14]. Upon binding of CD80 and CD86 to their receptor CD28, T cell activation and survival is promoted, whereas binding to the co-inhibitory receptor CTLA-4 T cell response is inhibited and regulates peripheral T cell tolerance [15]. Interestingly, monocyte-derived dendritic cells (DCs) from the blood of patients suffering from cardiovascular disease displayed an increased expression of CD80 and CD86 [3]. Moreover, in the human atherosclerotic plaque, CD80 and CD86 were expressed on a small proportion of T cells, macrophages, and other antigen-presenting cells (APCs), and were associated with plaque inflammation [14, 16]. Less data are available on the differential role of CD28 and CTLA-4 in vascular biology.

However, so far a non-invasive method allowing for a distinction of vulnerable from stable plaques is still lacking. The distinction would be of high benefit for the patient as it would allow surgical or other therapeutic intervention in case of a vulnerable plaque. Accordingly, a principal object of the present invention is to provide the means for such a non-invasive method.

### Summary of the Invention

According to the present invention, there is provided a compound for use in diagnosis or therapy of vulnerable atherosclerotic plaques in a human patient, said compound comprising an active moiety capable of binding to CD80 and/or CD86, said compound further containing a radioisotope.

By virtue of their specific binding to CD80 and/or CD86, such compounds are selectively accumulated in vulnerable atherosclerotic plaques and thus allow detection or treatment thereof by means of a suitable radioisotope contained in the respective compound.

In the present context and as customary in the field, a compound containing a radioisotope shall be understood as any compound wherein one specific nuclide has been replaced by a radioactive isotope thereof.

Advantageous embodiments are defined in the dependent claims.

According to one embodiment, the active moiety is capable of binding to CD80 (claim 2). According to another embodiment, the active moiety is capable of binding to CD86 (claim 3).

According to an embodiment aimed at diagnosis of unstable atherosclerotic plaques, the radioisotope is selected from the group consisting of ¹¹C, ¹³N, ¹⁵O, ¹³O, ¹²⁴I, ¹²³I, ¹⁸F ⁶⁶Ga, ⁶⁸Ga, ⁴⁴Sc, ⁷²As, ⁶⁰Cu, ⁶¹Cu, ⁶²Cu, ⁶⁴Cu, ¹⁹⁸Pb, ¹⁹⁷Hg, ⁹⁷Ru, ⁵²Fe, ⁵⁵Co, ⁸²Rb, ⁸²Sr, ⁶⁸Ge, ⁸⁹Zr, ⁸⁶Y, ^{99m}Tc, ¹¹¹In, ¹²⁵I, ⁴⁴Ti, ²⁰³Pb, ²⁰¹Tl, ⁶⁷Cu and ⁶⁷Ga (claim 4). Such isotopes are particularly useful for PET (positron emission tomography) or SPECT (single photon emission computed tomography). Such compounds have the potential to provide the clinics a non-invasive radiodiagnostic tool for imaging exclusively vulnerable plaques. Depending on the radioisotope, the compound can be distributed to all the PET centers within a certain geographic region. The clinicians can decide after a PET scan whether the patient needs to undergo surgery, an alternative therapeutic intervention or whether the patient is at low risk for plaque rupture and thus needs no intervention. As a result, health care resources can be allocated more efficiently and costs are reduced. According to one aspect of the invention, a method of imaging atherosclerotic vessels of a patient comprises administering to said patient a diagnostically effective amount of a compound as defined in this paragraph.

A further application involves the labeling with a different isotope for radiotherapy to harm the vulnerable plaques by use of a radioisotope which characteristics are high energy radiation and short radiation range. This would allow treating all vulnerable plaques at once without surgery. Therefore, in an embodiment aimed at therapy of unstable atherosclerotic plaques, the radioisotope is selected from the group consisting of ¹³¹I, ¹⁷⁷Lu, ⁹⁰Y, ¹⁶¹Tb, ⁶⁷Cu, ¹⁸⁸Re, ¹⁸⁶Re, ⁶⁷Ga, ¹⁵³Gd, ³²P, ²¹³Bi and ²²⁵Ac (claim 5). According to one aspect of the invention, a method of treating a patient suffering from unstable atherosclerotic plaques comprises administering to said patient a therapeutically effective amount of a compound as defined in this paragraph.

In a preferred embodiment, the compound is a so-called "small molecule" in the sense used in pharmacology and biochemistry, i.e. an organic compound with a molecular weight not exceeding about 800 Daltons. Small molecules have the advantage of comparatively rapid distribution within the body and thus are capable of reaching the desired target rapidly. This is a desirable property for diagnostic and therapeutic methods relying on radioisotopes.

According to a particularly advantageous embodiment (claim 6), the compound is selected from the group consisting of the compounds with general formula (I) 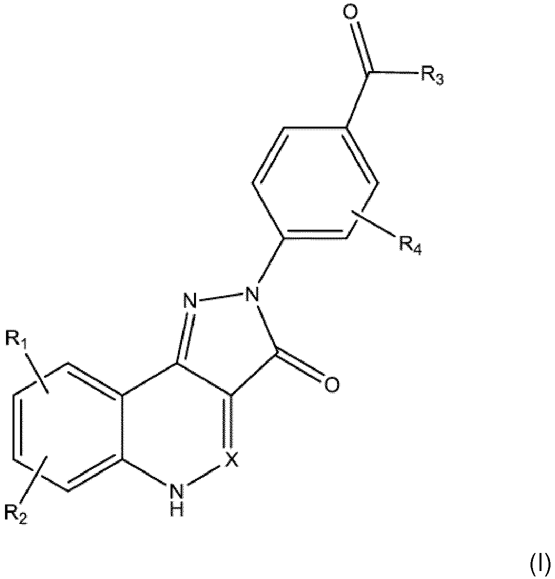 wherein:
- R1 is hydrogen, hydroxyl, a 5-homo- or heteroaromatic ring, a 6-homo- or heteroaromatic ring, a C1-4 alkyl, a C1-4 alkoxy, a fluoro C1-4 alkoxy or a carboxylic acyloxy group, or a halogen selected from F, Cl, Br and I,
- R2 is hydrogen, hydroxyl, a 5-homo- or heteroaromatic ring, a 6-homo- or heteroaromatic ring, a C1-4 alkyl, a C1-4 alkoxy, a fluoro C1-4 alkoxy or a carboxylic acyloxy group, or a halogen selected from F, Cl, Br and I;
- R3 is hydroxyl, a 5-homo- or heteroaromatic ring, a 5-homo- or heteroaliphatic ring, a 6-homo- or heteroaromatic ring, a 6-homo- or heteroaliphatic ring, a C1-4 alkyl, a C1-4 alkoxy, a fluoro C1-4 alkoxy , a carboxylic acyloxy group, a 5-cyclo-amine, a 6-cyclo-amine or an alkyl amine;
- R4 is fluorine or hydrogen;
- or wherein any one of said R1, R2, R3 and R4 is a chelator optionally provided with a linker group, said chelator being selected from the group consisting of DOTA, DO3A, M4DOTA, M4DO3A, DOTMA, DO3MA, M4DOTMA, MPDO3A, NOTA, NODAGA and TRAP, said chelator being chelated to said radioisotope, said radioisotope being a metallic ion;
- and wherein X is nitrogen or carbon.

In certain embodiments, any one of said substituents R1, R2, R3 and R4 is a chelator, i.e. a compound that is capable of chelating, or binding, a metal ion.

The chelators mentioned above with their acronyms are generally known and listed hereinbelow:

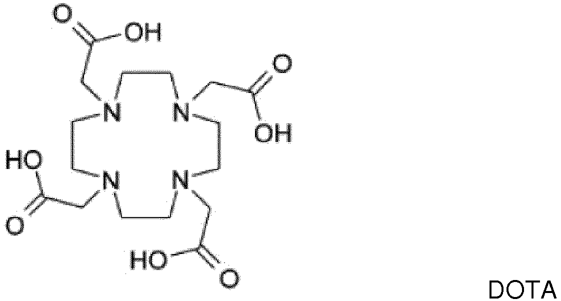

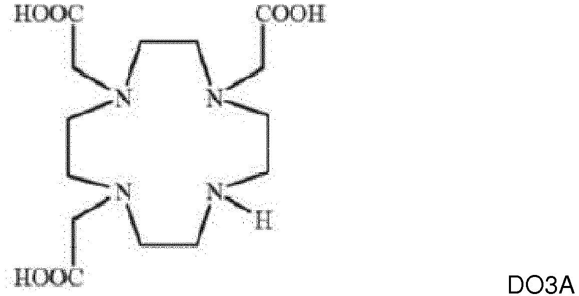

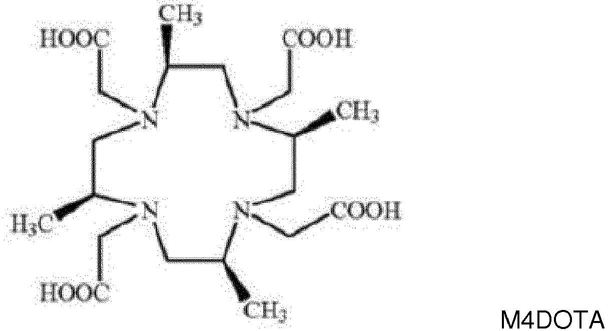

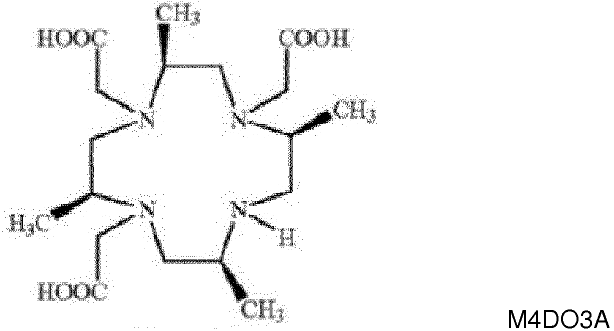

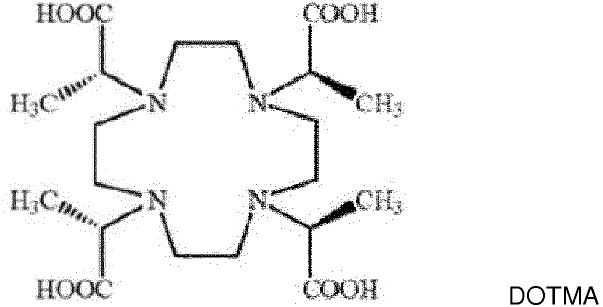

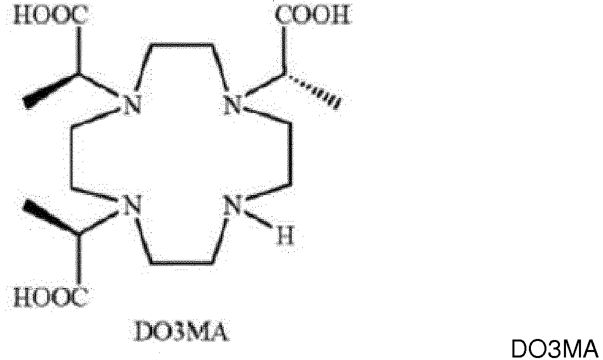

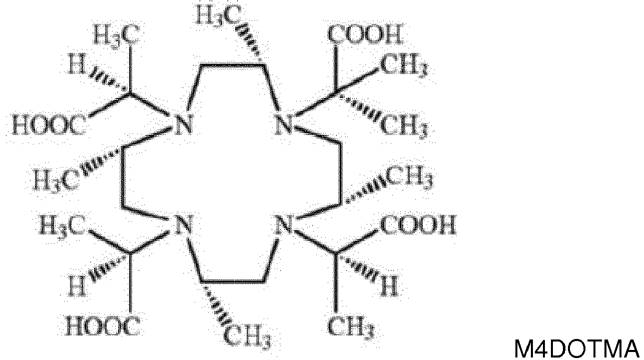

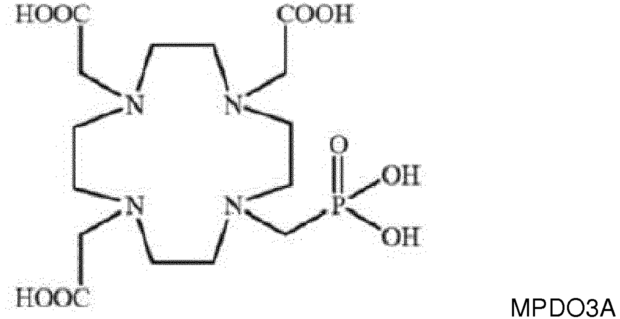

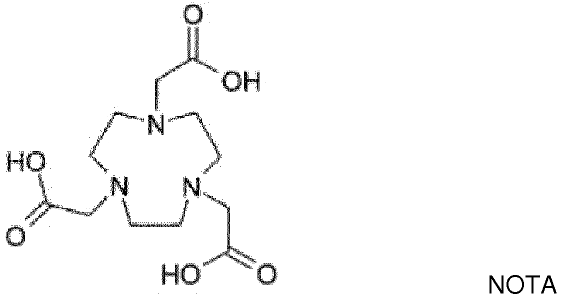

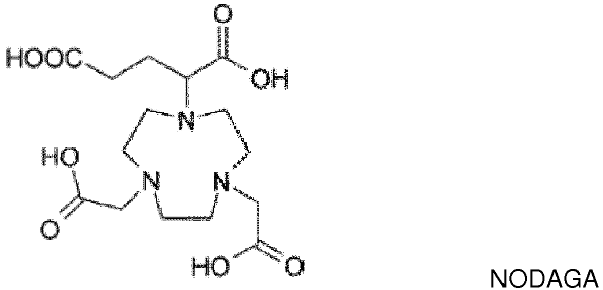

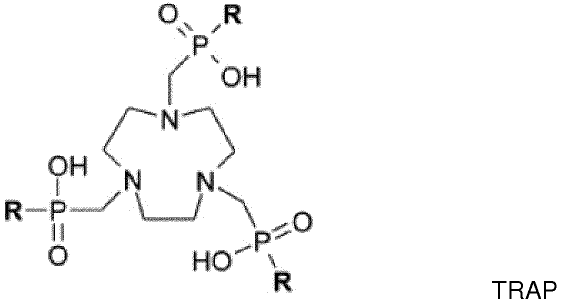

In certain embodiments, a linker group is arranged between the chelator and the ring skeleton or terminal carbonyl group of compound (I). The linker may be of any type known to the skilled person. The linker may be initially attached to the chelator or to the targeting ligand. A linker may be attached to the chelator, while another linker is attached to the targeting ligand, such that the two linkers may then be joined. According to a preferred embodiment, the linker group is a PEG (polyethylene glycol)-, alkyl- or peptidic spacer (claim 7).

Huxley et al. have reported a series of small compounds (average MW 424 ± 75) falling under the above definition [2, 3]. These compounds are generally suitable for radiolabeling, e.g. with ¹⁸F or ¹¹C.

In a particularly preferred "small molecule" embodiment (claim 8), R1 is hydrogen, R2 is fluorine, R3 is N-¹¹C-methylpiperidine-4-amino, R4 is hydrogen, and X is nitrogen. This compound will henceforth be denoted as "Compound 4" in accordance with Huxley's work.

Although peptides and proteins have certain disadvantages as ligands for PET imaging due to their larger size, they are nonetheless considered in the realm or the present invention in view of their potentially high selectivity.

An immunoglobulin-fusion protein construct of CTLA-4, named LEA29Y (belatacept, Nulojix®) has been characterized as immunosuppressant in the context of organ transplantation in primates. Both LEA29Y and a 13.9 kDa truncated CTLA-4 peptide bind with high affinity to CD86/CD80. These peptides are starting points for the development of alternative PET/SPECT probes for vulnerable plaques.

Therefore, according to a further embodiment, the active moiety comprises the extracellular domain, or part of the extracellular domain, of radiolabelled CTLA-4 or an affinity-maturated analogue thereof (claim 9).

In a further embodiment, the active moiety comprises radiolabelled sCTLA-4, or part thereof, or an affinity-maturated analogue (claim 10).

In a still further embodiment, the compound is an antibody or an antibody fragment containing the extracellular domain or part of the extracellular domain of radiolabelled CTLA-4 or an affinity-maturated analogue thereof (claim 11).

In a preferred embodiment, the compound is radiolabeled LEA29Y (claim 12).

In yet another embodiment, the active moiety comprises the extracellular domain, or part of the extracellular domain, of CD28 or an affinity-maturated analogue thereof (claim 13).

### Brief description of the drawings

The above mentioned and other features and objects of this invention and the manner of achieving them will become more apparent and this invention itself will be better understood by reference to the following description of various embodiments of this invention taken in conjunction with the accompanying drawings, wherein:

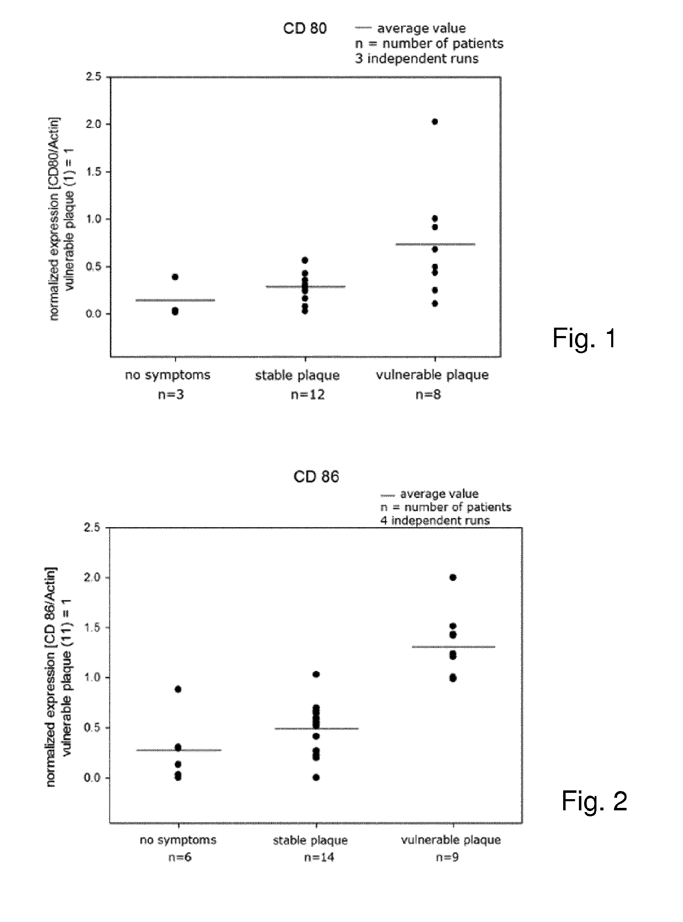

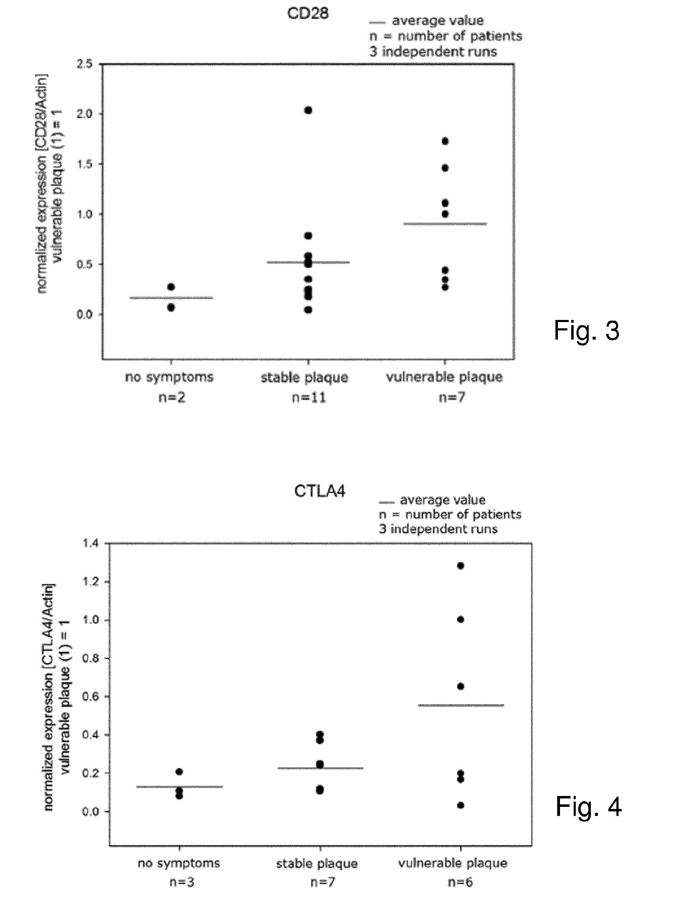

- Figs. 1 to 4: show real time-PCR (RNA expression levels) verification of CD80 (Fig. 1), CD86 (Fig. 2), CD28 (Fig. 3) and CTLA4 (Fig. 4) gene expression in human derived atherosclerotic stable plaques, vulnerable plaques and in artery tissue from asymptomatic patients (no symptoms); and

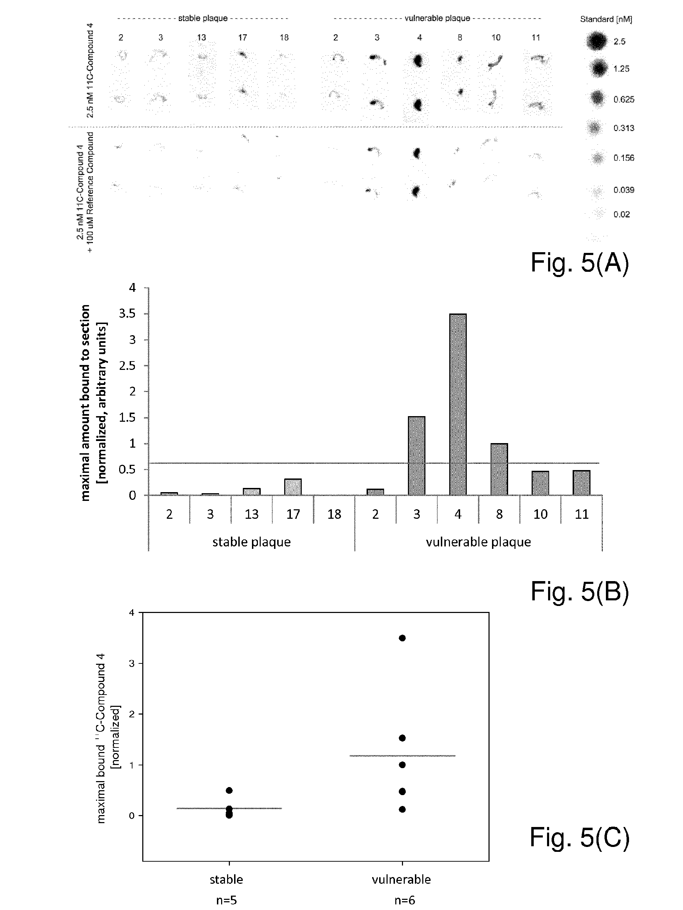

- Fig. 5: shows autoradiographies on human atherosclerotic plaques with 2.5 nM ¹¹C-Compound 4: (A) representative slices of human stable and vulnerable plaques without (upper panel) and with (lower panel) blocker (100 µM reference substance); (B) and (C) semiquantitative evaluation of maximal bound ¹¹C-Compound 4; values are normalized to vulnerable plaque 8. The red line indicates the average value (in (B) 0.7; in (C) stable = 0.15, vulnerable = 1.2).

### Detailed description of the invention

Human atherosclerotic plaques from Arteria carotis provided by the University Hospital Zürich have been characterized by a surgeon/pathologist to be either stable or vulnerable. By using quantitative real time PCR, an increased expression of CD80 (significant), CD86 (significant), CD28 (not significant) and CTLA-4 (not significant) was found in vulnerable plaques compared to stable plaques (see Figs. 1 to 4).

A selection of compounds known to be capable of binding to CD80 and/or CD86 was radiolabeled for subsequent characterization as illustrated below.

The following examples illustrate the invention.

### Example 1: Preparation of [¹¹C]-radiolabeled "Compound 4"

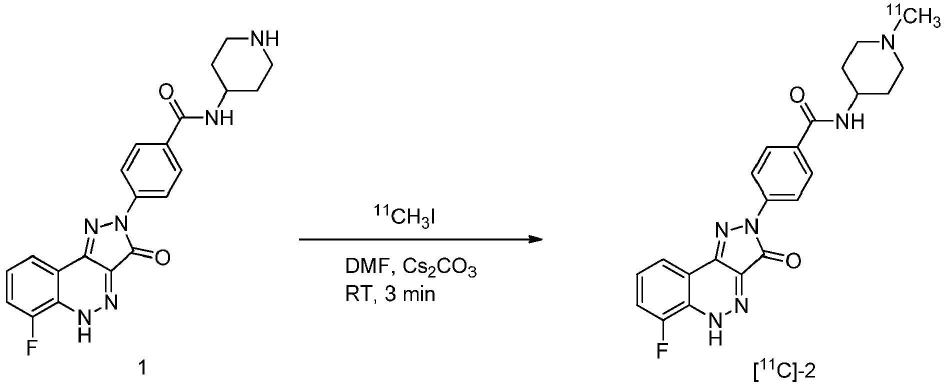

Carbon-11 was produced via the ¹⁴N(p,α)¹¹C nuclear reaction at a Cyclone 18/9 cyclotron (18-MeV, IBA, Belgium) in the form of [¹¹C]-CO₂. [¹¹C]-Methyl iodide ([¹¹C]-Mel) was generated in a 2-step reaction sequence involving the catalytic reduction of [¹¹C]-CO₂ to [¹¹C]-methane and subsequent gas phase iodination. Radiolabeling precursor compound **1** (4-(6-fluoro-3-oxo-3,5-dihydro-2H-pyrazolo[4,3-c]cinnolin-2-yl)-N-(piperidin-4-yl)benzamide) can be readily prepared according to published methods in patent WO2004081011 (Matthews, I.R. September 23, 2004. Avidex Ltd., United Kingdom. Immunomodulating heterocyclic compounds. PCT Int. Appl. WO 2004/081011).

Radiolabeled "Compound 4", i.e. [¹¹C]-**2** was prepared by reaction of compound **1** (0.5-1 mg) with [¹¹C]-Mel in DMF solution in the presence of Cs₂CO₃ (5 mg) at room temperature for 3 min (Scheme 1). The crude product was diluted with 25% MeCN in water (1.5 ml) and injected onto semi- preparative high-performance liquid chromatography (HPLC) (ACE column, 7.8 x 50 mm, 5 µ; mobile phases, A: 50 mM NaOAc and B: acetonitrile with the following gradient: 0-3 min, 10% B; 3.0-13 min, 10-45% B; 13-14 min 45-10% B; 14-18 min, 10%B, flow rate, 4 mL/min). The radiolabeled product was collected, diluted with water (10 mL), passed through a preconditioned C18 cartridge (Waters), washed with water (5 mL), and eluted the product with EtOH (0.5 mL). The product [¹¹C]-**2** was diluted with water (9.5 mL) and used for all in vitro/in vivo studies.

### Example 2: Binding study with [¹¹C]-radiolabeled "Compound 4"

Compound 4 was labeled with ¹¹C (specific activity ~350 GBq/µmol) and human atherosclerotic sections were incubated with 2.5 nM for 20 min, room temperature. Nonspecific binding was determined in the presence of excess nonradioactive Compound 4 (100 µM). After washing to remove unspecific bound ¹¹C-Compound 4, slides were exposed to a radiosensitive film (BAS-5000 Fujifilm) for 30 min. Calibration standards (2.5 - 0.02 nM) were included in the cassette. Films were developed using an automatic film processor (BAS 5000, Fuji) Quantitative analysis was performed using a computerized image analysis system (AIDA Image Analyzer Software). Optical density readings, corrected for background, were made and values standardized against the linear portion of a curve generated using standards and expressed as maximal bound ¹¹C-Compound 4. Values were normalized to patient "vulnerable plaque 8" (Fig. 5).

These results show that ¹¹C-Compound 4 binds with a higher affinity to vulnerable plaques compared to stable plaques.

### References

1. Yazdani, S.K., et al., Pathology and vulnerability of atherosclerotic plaque: identification, treatment options, and individual patient differences for prevention of stroke. Current treatment options in cardiovascular medicine, 2010. 12(3): p. 297-314.
2. Huxley, P., et al., High-affinity small molecule inhibitors of T cell costimulation: compounds for immunotherapy. Chemistry and Biology, 2004. 11 (12): p. 1651-8.
3. Dopheide, J.F., et al., Monocyte-derived dendritic cells of patients with coronary artery disease show an increased expression of costimulatory molecules CD40, CD80 and CD86 in vitro. Coronary Artery Disease, 2007. 18(7): p. 523-31.
4. Vancraeynest, D., et al., Imaging the vulnerable plaque. Journal of the American College of Cardiology, 2011. 57(20): p. 1961-79.
5. Torzewski, M., et al., Reduced in vivo aortic uptake of radiolabeled oxidation-specific antibodies reflects changes in plaque composition consistent with plaque stabilization. Arteriosclerosis, thrombosis, and vascular biology, 2004. 24(12): p. 2307-12.
6. Fujimoto, S., et al., Molecular imaging of matrix metalloproteinase in atherosclerotic lesions: resolution with dietary modification and statin therapy. Journal of the American College of Cardiology, 2008. 52(23): p. 1847-57.
7. Kolodgie, F.D., et al., Targeting of apoptotic macrophages and experimental atheroma with radiolabeled annexin V: a technique with potential for noninvasive imaging of vulnerable plaque. Circulation, 2003. 108(25): p. 3134-9.
8. Kietselaer, B.L., et al., Noninvasive detection of plaque instability with use of radiolabeled annexin A5 in patients with carotid-artery atherosclerosis. The New England journal of medicine, 2004. 350(14): p. 1472-3.
9. Sarai, M., et al., Broad and specific caspase inhibitor-induced acute repression of apoptosis in atherosclerotic lesions evaluated by radiolabeled annexin A5 imaging. Journal of the American College of Cardiology, 2007. 50(24): p. 2305-12.
10. Hartung, D., et al., Radiolabeled Monocyte Chemotactic Protein 1 for the detection of inflammation in experimental atherosclerosis. Journal of nuclear medicine : official publication, Society of Nuclear Medicine, 2007. 48(11): p. 1816-21.
11. Nahrendorf, M., et al., 18F-4V for PET-CT Imaging of VCAM-1 Expression in Atherosclerosis. JACC: Cardiovascular Imaging, 2009. 2(10): p. 1213-1222.
12. Laitinen, I., et al., Evaluation of alphavbeta3 integrin-targeted positron emission tomography tracer 18F-galacto-RGD for imaging of vascular inflammation in atherosclerotic mice. Circulation. Cardiovascular imaging, 2009. 2(4): p. 331-8.
13. Gerdes, N. and A. Zirlik, Co-stimulatory molecules in and beyond costimulation - tipping the balance in atherosclerosis? Thrombosis and Haemostasis, 2011. 106(5).
14. de Boer, O.J., et al., Costimulatory molecules in human atherosclerotic plaques: an indication of antigen specific T lymphocyte activation. Atherosclerosis, 1997. 133(2): p. 227-34.
15. Zang, X. and J.P. Allison, The B7 family and cancer therapy: costimulation and coinhibition. Clinical cancer research : an official journal of the American Association for Cancer Research, 2007. 13(18 Pt 1): p. 5271-9.
16. Kim, W.J., et al., Comparative analysis of the expression patterns of various TNFSF/TNFRSF in atherosclerotic plaques. Immunological Investigations, 2008. 37(4): p. 359-73.

## Claims

1. A compound for use in diagnosis or therapy of vulnerable atherosclerotic plaques in a human patient, **characterized in that** said compound comprises an active moiety capable of binding to CD80 and/or CD86 and that said compound contains a radioisotope.

2. The compound according to claim 1, wherein said active moiety is capable of binding to CD80.

3. The compound according to claim 1, wherein said active moiety is capable of binding to CD86.

4. The compound according to any one of claims 1 to 3, wherein said radioisotope is selected from the group consisting of ¹¹C, ¹³N, ¹⁵O, ¹³O, ¹²⁴I, ¹²³I, ¹⁸F, ⁶⁶Ga, ⁶⁸Ga, ⁴⁴Sc, ⁷²As, ⁶⁰Cu, ⁶¹Cu, ⁶²Cu, ⁶⁴Cu, ¹⁹⁸Pb, ¹⁹⁷Hg, ⁹⁷Ru, ⁵²Fe, ⁵⁵Co, ⁸²Rb, ⁸²Sr, ⁶⁸Ge, ⁸⁹Zr, ⁸⁶Y, ^{99m}Tc, ¹¹¹In, ¹²⁵I, ⁴⁴Ti, ²⁰³Pb, ²⁰¹Tl, ⁶⁷Cu and ⁶⁷Ga for use in diagnosis.

5. The compound according to any one of claims 1 to 3, wherein said radioisotope is selected from the group consisting of ¹³¹I, ¹⁷⁷Lu, ⁹⁰Y, ¹⁶¹Tb, ⁶⁷Cu, ¹⁸⁸Re, ¹⁸⁶Re_{,} ⁶⁷Ga_{,} ¹⁵³Gd_{,} ³²P, ²¹³Bi and ²²⁵Ac for use in therapy.

6. The compound according to any one of claims 1 to 5, selected from the group consisting of the compounds with general formula (I) 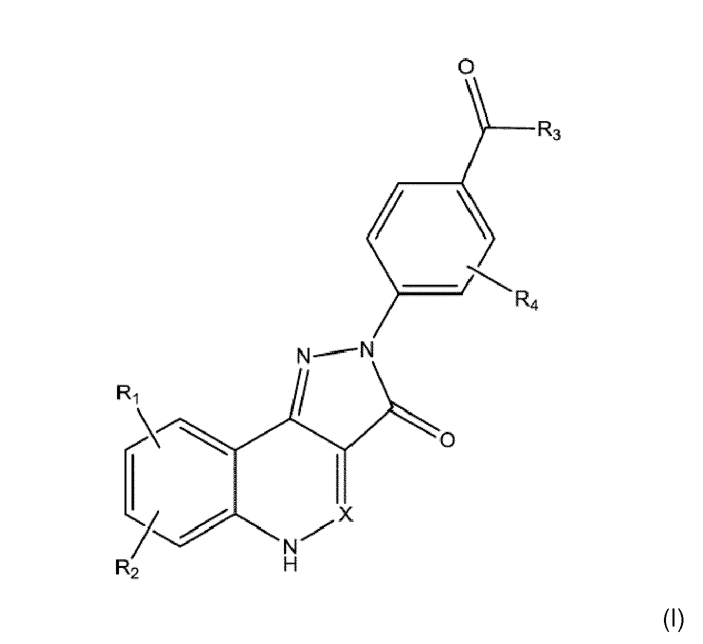 wherein:
R1 is hydrogen, hydroxyl, a 5-homo- or heteroaromatic ring, a 6-homo- or heteroaromatic ring, a C1-4 alkyl, a C1-4 alkoxy, a fluoro C1-4 alkoxy or a carboxylic acyloxy group, or a halogen selected from F, Cl, Br and I,
R2 is hydrogen, hydroxyl, a 5-homo- or heteroaromatic ring, a 6-homo- or heteroaromatic ring, a C1-4 alkyl, a C1-4 alkoxy, a fluoro C1-4 alkoxy or a carboxylic acyloxy group, or a halogen selected from F, Cl, Br and I;
R3 is hydroxyl, a 5-homo- or heteroaromatic ring, a 5-homo- or heteroaliphatic ring, a 6-homo- or heteroaromatic ring, a 6-homo- or heteroaliphatic ring, a C1-4 alkyl, a C1-4 alkoxy, a fluoro C1-4 alkoxy or a carboxylic acyloxy group, a 5-cyclo-amine, a 6-cyclo-amine or a alkyl amine;
R4 is fluorine or hydrogen;
or wherein any one of said R1, R2, R3 and R4 is a chelator optionally provided with a linker group, said chelator being selected from the group consisting of DOTA, DO3A, M4DOTA, M4DO3A, DOTMA, DO3MA, M4DOTMA, MPDO3A, NOTA, NODAGA and TRAP, said chelator being chelated to said radioisotope, said radioisotope being a metallic ion;
and wherein X is nitrogen or carbon.

7. The compound according to claim 6, wherein said linker group is a PEG (polyethylene glycol)-, alkyl- or peptidic spacer.

8. The compound according to claim 6 or 7, wherein R1 is hydrogen, R2 is fluorine, R3 is N-¹¹C-methylpiperidine-4-amino, R4 is hydrogen, and X is nitrogen.

9. The compound according to claim 1, wherein said active moiety comprises the extracellular domain, or part of the extracellular domain, of radiolabelled CTLA-4 or an affinity-maturated analogue thereof.

10. The compound according to claim 1, wherein said active moiety comprises radiolabelled sCTLA-4, or part thereof, or an affinity-maturated analogue.

11. The compound according to claim 1, wherein said compound is an antibody or an antibody fragment containing the extracellular domain or part of the extracellular domain of radiolabelled CTLA-4 or an affinity-maturated analogue thereof.

12. The compound according to claim 1, wherein said compound is radiolabeled LEA29Y.

13. The compound according to claim 1, wherein said active moiety comprises the extracellular domain, or part of the extracellular domain, of CD28 or an affinity-maturated analogue thereof.

14. A method of imaging atherosclerotic vessels of a patient, comprising administering to said patient a diagnostically effective amount of a compound according to claim 4.

15. A method of treating a patient suffering from unstable atherosclerotic plaques, comprising administering to said patient a therapeutically effective amount of a compound according to claim 5.
